# EUROPEAN PATENT APPLICATION

(11) **EP 4 725 519 A1**
(43) Date of publication of application: **15.04.2026**
(21) Application number: 24864987.3
(22) Date of filing: 15.05.2024
(51) Int. Cl.: A61M 5/142, A61M 5/158

(54) **PHARMACEUTICAL LIQUID ADMINISTRATION DEVICE**

(30) Priority: 12.09.2023 JP 2023147799
(71) Applicant: Terumo Kabushiki Kaisha, Tokyo 151-0072 (JP)
(72) Inventor: YANAGISAWA, Katsuya, Ashigarakami-gun, Kanagawa 259-0151 (JP); HASEGAWA, Makoto, Ashigarakami-gun, Kanagawa 259-0151 (JP)
(74) Representative: KIPA AB
(86) International application number: PCT/JP2024/017966
(87) International publication number: WO 2025/057479

(57) **Abstract**

A liquid medicine administration device (10) includes: a puncture structure (14); a needle cover (18) provided movably with respect to a housing (12); a sliding member (20) that is provided slidably with respect to the housing (12) and is coupled to the needle cover (18) via a coupling portion (19); and an adhesive member (22) fixed to the sliding member (20). In a case where the needle cover (18) is located at a puncture permission position, movement of the needle cover (18) to a shielding position is restricted by a first lock portion (210) of the sliding member (20). When the housing (12) is separated from the skin (S) of the living body, the sliding member (20) is movable to a post-sliding position, and the needle cover (18) is movable to the shielding position.

## Description

### Technical Field

The present invention relates to a liquid medicine administration device.

### Background Art

Conventionally, a liquid medicine administration device including a needle cover for covering a puncture needle after liquid medicine administration is known (for example, see JP 2020-522315 A below).

### Summary of Invention

There is a demand for a liquid medicine administration device in which a state of a needle tip of a puncture needle that is not covered with the needle cover is maintained in an initial state, the needle cover is movable after administration of a liquid medicine, and the needle tip of the puncture needle can be covered with the needle cover.

It is therefore an object of the present invention to solve the above-described problem.

(1) An aspect of the present invention is a liquid medicine administration device including: a puncture structure that is capable of puncturing a skin of a living body and has a puncture needle for administering a liquid medicine; a housing that accommodates the puncture structure and has a bottom wall portion which enables the puncture needle to project; a needle cover provided movably with respect to the housing; a sliding member that faces the bottom wall portion of the housing and is provided slidably with respect to the housing; and an adhesive member that is fixed to the sliding member and has an adhesive surface which is capable of adhering to the skin of the living body. The needle cover is movable from a puncture permission position at which the puncture needle is permitted to puncture the skin to a shielding position at which a needle tip of the puncture needle is covered. The needle cover and the sliding member are coupled via a coupling portion to be linked to each other. The sliding member has a first lock portion that engages with a first engagement portion provided in the housing. In a case where the needle cover is located at the puncture permission position, the sliding member is located at a pre-sliding position, at which the sliding member is fixed to the housing by the first lock portion, and restricts the needle cover from moving to the shielding position. When the housing is separated from the skin of the living body, the first engagement portion and the first lock portion are disengaged, so that the sliding member is movable to a post-sliding position and the needle cover is movable to the shielding position.

According to this liquid medicine administration device, since the movement of the needle cover to the shielding position is restricted by the first lock portion in the initial state, the needle tip of the puncture needle is not covered with the needle cover, and the puncture of the skin of the living body can be performed. On the other hand, since movement restriction of the needle cover by the first lock portion is cancelled by separating the housing from the skin of the living body after administration of the liquid medicine, the needle tip of the puncture needle can be covered with the needle cover.

(2) In the liquid medicine administration device according to (1), a part of the adhesive member may be fixed to the first lock portion, and when the housing is separated from the skin of the living body, the first lock portion may be drawn by the skin to be separated from the bottom wall portion of the housing and disengaged from the first engagement portion.

This enables, in a simple structure, a motion of the housing that is separated from the skin of the living body and disengagement of the first lock portion and the first engagement portion from each other to be linked to each other.

(3) In the liquid medicine administration device according to (2), the sliding member may have a sliding plate coupled to the needle cover via the coupling portion, the first lock portion may be a movable piece provided movably with respect to the sliding plate, and a part of a circumferential edge portion of the first lock portion may be coupled to the sliding plate.

According to this configuration, since the first lock portion is provided movably with respect to the sliding plate, the first lock portion can be separated from the first engagement portion in a simple configuration when the housing is separated from the skin of the living body.

(4) In the liquid medicine administration device according to any one of (1) to (3), the sliding member may have a second lock portion that is engageable with a second engagement portion provided in the housing, and in a case where the sliding member is located at the post-sliding position, the second lock portion may engage with the second engagement portion and restrict the sliding member from moving toward the pre-sliding position.

According to this configuration, since the sliding member is fixed to the post-sliding position by the second lock portion, the needle cover can be prevented from returning to the puncture permission position.

(5) In the liquid medicine administration device according to (4), the sliding member may have a sliding plate coupled to the needle cover via the coupling portion, and the second lock portion may be an elastic piece having one end coupled to the sliding plate.

This enables, in a simple structure, the second lock portion to engage with the second engagement portion of the housing in a case where the sliding member reaches the post-sliding position.

(6) In the liquid medicine administration device according to (5), the second lock portion may have an engagement claw portion having an inclined surface inclined with respect to a moving direction of the sliding member, the housing may have an accommodation portion formed by a groove or a hole that accommodates the engagement claw portion in a case where the sliding member is located at the pre-sliding position, the second engagement portion may be a groove or a hole that engages with the engagement claw portion in a case where the sliding member is located at the post-sliding position, and when the sliding member moves from the pre-sliding position toward the post-sliding position, the inclined surface may come into sliding contact with the accommodation portion, and the second lock portion may be deformed in a direction away from the accommodation portion, so that the engagement claw portion may be detached from the accommodation portion.

According to this configuration, since the engagement claw portion of the second lock portion has the inclined surface, the engagement claw portion can be smoothly detached from the accommodation portion.

(7) In the liquid medicine administration device according to (6), the adhesive member may not be fixed to the second lock portion.

According to this configuration, the second lock portion can be prevented from being drawn and deformed by the skin when the adhesive member is detached from the skin.

(8) In the liquid medicine administration device according to any one of (1) to (7), the needle cover may have a shape of a lid-shaped member which partially covers the housing, may be in a lid closed state at the puncture permission position, and may be in a lid open state at the shielding position, and the needle cover may have an opening portion that permits reception of the puncture structure in the lid closed state, and a cover wall that covers the needle tip of the puncture needle in the lid open state.

According to this configuration, the needle cover that does not inhibit activation of the puncture structure in the case of the lid closed state and suitably covers the needle tip of the puncture needle in the case of the lid open state can be realized in a simple structure.

(9) In the liquid medicine administration device according to (8), the puncture structure may have an abutting member having an abutting surface that comes into contact with the skin, the abutting member may be provided movably with respect to the housing to enable the abutting surface to project from the bottom wall portion, in a case where the needle cover is located at the puncture permission position, the opening portion may permit the abutting member to be received, the puncture needle may be relatively movable with respect to the abutting member and may be capable of projecting from the abutting surface toward the skin, and the abutting member may relatively move with respect to the housing to maintain contact between the abutting surface and the skin when relative positions of the housing and a portion of the skin which faces the abutting surface change in a state where the housing is placed on the skin.

According to this configuration, since the abutting member relatively moves with respect to the housing to follow the skin even in a case where a relative position between the housing and the skin changes due to a body movement of the living body in a state where the skin of the living body is punctured by the puncture needle, the puncture needle is appropriately prevented from being pulled out of the skin.

(10) In the liquid medicine administration device according to (8), the needle cover may have a notch portion that communicates with the opening portion, and a part of the puncture structure may pass through the notch portion when the needle cover moves from the puncture permission position to the shielding position.

According to this configuration, the needle cover can be moved to the shielding position without any hindrance by providing the notch portion while an area of a wall portion of the needle cover is sufficiently secured to suitably cover the needle tip of the puncture needle.

(11) The liquid medicine administration device according to any one of (1) to (10) may include a shielding biasing member that biases the needle cover toward the shielding position.

According to this configuration, the needle cover can be automatically moved to the shielding position by a biasing force of the shielding biasing member when the first lock portion and the first engagement portion are disengaged.

(12) In the liquid medicine administration device according to any one of (1) to (11), the needle cover, the coupling portion, and the sliding member may be formed as one component.

According to this configuration, an increase in the number of components can be reduced.

(13) In the liquid medicine administration device according to any one of (1) to (11), the adhesive member may be fixed to the needle cover and the sliding member, and the coupling portion may be a part of the adhesive member.

According to the liquid medicine administration device of the present invention, the needle tip of the puncture needle is not covered with the needle cover, and the puncture of the skin of the living body can be performed. On the other hand, since the movement restriction of the needle cover by the first lock portion is cancelled by separating the housing from the skin of the living body after the administration of the liquid medicine, the needle tip of the puncture needle can be covered with the needle cover.

### Brief Description of Drawings

Fig. 1 is a perspective view of a liquid medicine administration device according to an embodiment of the present invention.
Fig. 2 is an exploded perspective view of the liquid medicine administration device.
Fig. 3 is a cross-sectional view of the liquid medicine administration device in an initial state.
Fig. 4 is an exploded perspective view of a puncture structure.
Fig. 5 is a perspective view of the liquid medicine administration device from an adhesive member side.
Fig. 6A is a cross-sectional view illustrating a relationship between a first lock portion and a first engagement portion in a case where a sliding member is located at a pre-movement position. Fig. 6B is a cross-sectional view illustrating a relationship between the first lock portion and the first engagement portion in a case where the sliding member is located at a post-movement position.
Fig. 7A is a cross-sectional view illustrating a relationship between a second lock portion and a second engagement portion in a case where the sliding member is located at the pre-movement position. Fig. 7B is a cross-sectional view illustrating a relationship between the second lock portion and the second engagement portion in a case where the sliding member is located at the post-movement position.
Fig. 8 is a cross-sectional view of the liquid medicine administration device in a state where an attachment is removed.
Fig. 9 is a view illustrating a state where the liquid medicine administration device adheres to a skin.
Fig. 10 is a cross-sectional view of the liquid medicine administration device in Fig. 9.
Fig. 11 is a cross-sectional view of the liquid medicine administration device in a state where the skin is punctured by a puncture needle.
Fig. 12 is a view illustrating a state when the liquid medicine administration device is detached from the skin.
Fig. 13 is a view illustrating a state where the needle cover covers the puncture needle.
Fig. 14 is a view illustrating a state where a relative position between the housing and the skin is changed by a body movement of the living body.
Fig. 15 is a perspective view illustrating a coupling portion according to a modification example.

### Description of Embodiments

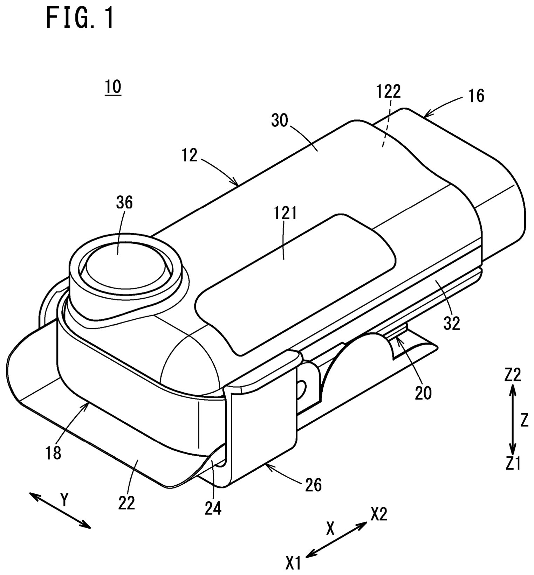

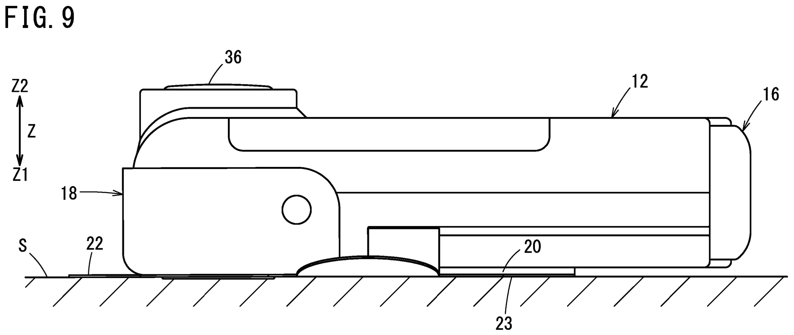

A liquid medicine administration device 10 illustrated in Fig. 1 is used to adhere to a skin S (refer to Fig. 9) of a patient which is a living body and administer a liquid medicine subcutaneously to the patient. The liquid medicine administration device 10 continuously administers the liquid medicine into the living body over a relatively long time (for example, about several minutes to several hours). The liquid medicine administration device 10 may intermittently administer the liquid medicine into the living body. Examples of the liquid medicine include protein preparations such as insulin preparations, narcotic analgesics, diuretics, and the like.

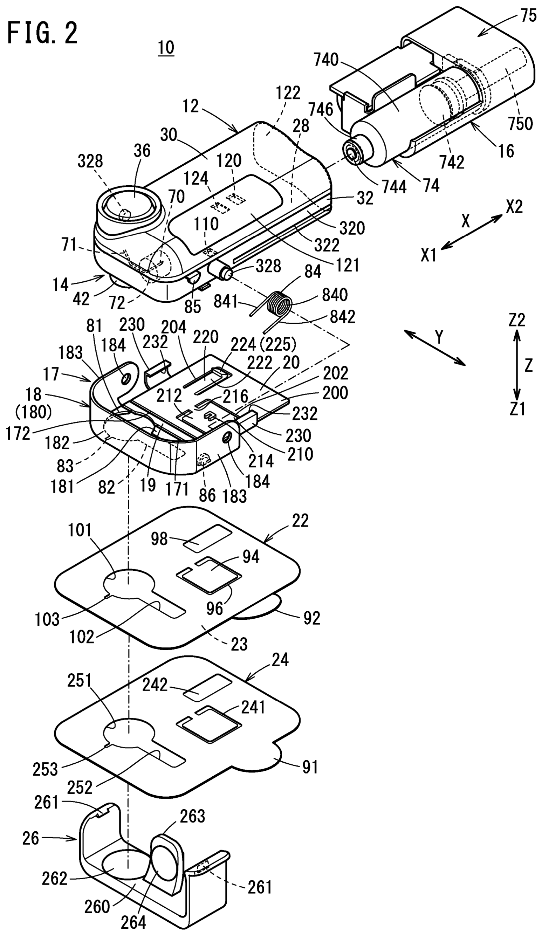

As illustrated in Fig. 2, the liquid medicine administration device 10 includes a housing 12, a puncture structure 14, a syringe pump 16, a needle cover 18, a sliding member 20, an adhesive member 22, a release sheet 24, and an attachment 26.

The housing 12 extends in one direction (X direction). The housing 12 has a bottom wall portion 28 constituting a wall portion on one side in a thickness direction (Z direction) of the housing 12, a ceiling portion 30 constituting a wall portion on the other side in the thickness direction of the housing 12, and a circumferential wall portion 32 connecting the bottom wall portion 28 and the ceiling portion 30. Hereinafter, regarding the liquid medicine administration device 10 and the components thereof, a Y direction orthogonal to the X direction and the Z direction is referred to as a "width direction".

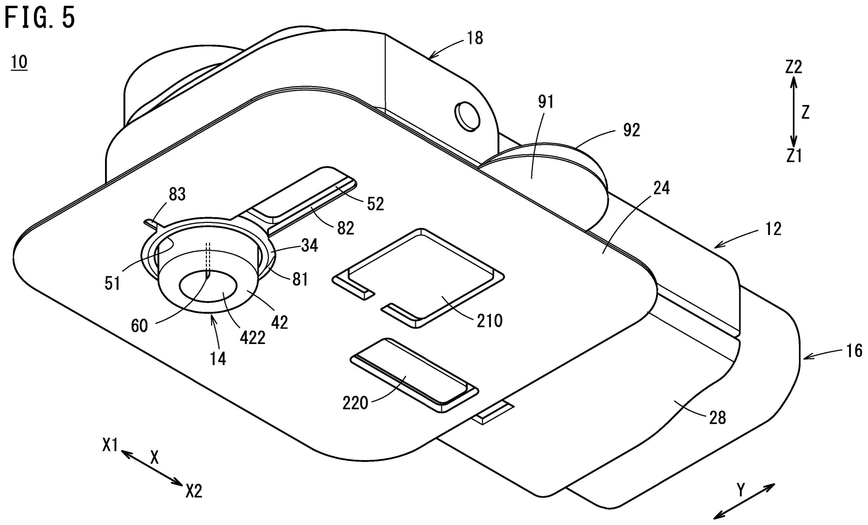

As illustrated in Fig. 5, a first opening portion 51 and a second opening portion 52 which penetrate in the Z direction are formed in the bottom wall portion 28 of the housing 12. The first opening portion 51 and the second opening portion 52 are positioned at one end portion (end portion in an X1 direction) of the bottom wall portion 28. The first opening portion 51 and the second opening portion 52 are arranged in the width direction of the housing 12.

The first opening portion 51 is a circular hole. The first opening portion 51 is a hole for allowing a part of the puncture structure 14 to project outward from the housing 12 toward the skin S of the living body. An annular protrusion 34 that surrounds the first opening portion 51 and protrudes in a puncture direction (Z1 direction) from the bottom wall portion 28 is provided in the housing 12. The second opening portion 52 is a hole for inserting a part (a projecting wall 263 to be described below) of the attachment 26 (refer to Fig. 2) into the inside from the outside of the housing 12.

As illustrated in Fig. 1, a window 121 is provided in a part of the ceiling portion 30. An insertion port 122 for inserting the syringe pump 16 into the housing 12 is provided at an end of the housing 12 in an X2 direction. An operation cover 36 is provided at an end of the ceiling portion 30 in the X1 direction. When the operation cover 36 is operated (pressed), the puncture structure 14 is activated.

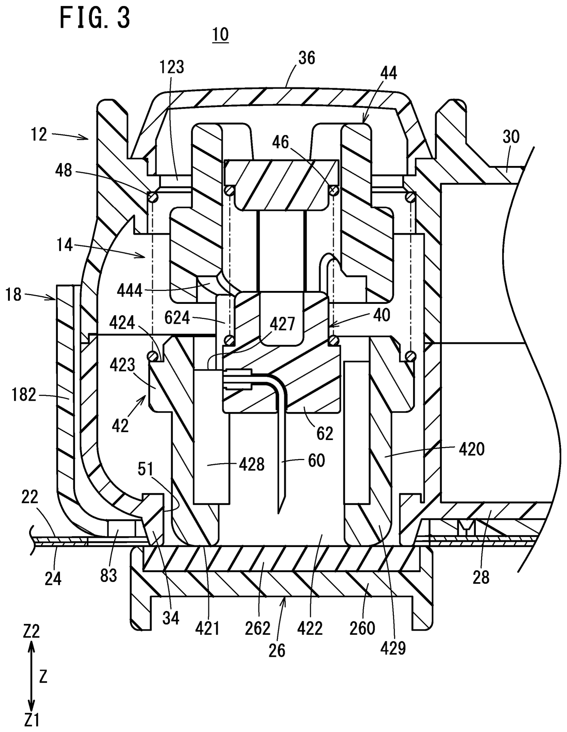

As illustrated in Fig. 3, a hole portion 123 is formed in the ceiling portion 30 of the housing 12. The hole portion 123 is covered with the operation cover 36 attached to the ceiling portion 30.

The puncture structure 14 is attached to the housing 12. The puncture structure 14 includes a needle member 40, an abutting member 42, a pusher 44, a puncture biasing member 46, and a following biasing member 48. Hereinafter, regarding the puncture structure 14 and the components thereof, the puncture direction (Z1 direction) of the needle member 40 into the skin S is referred to as a distal direction, and a direction opposite to the puncture direction is referred to as a proximal direction.

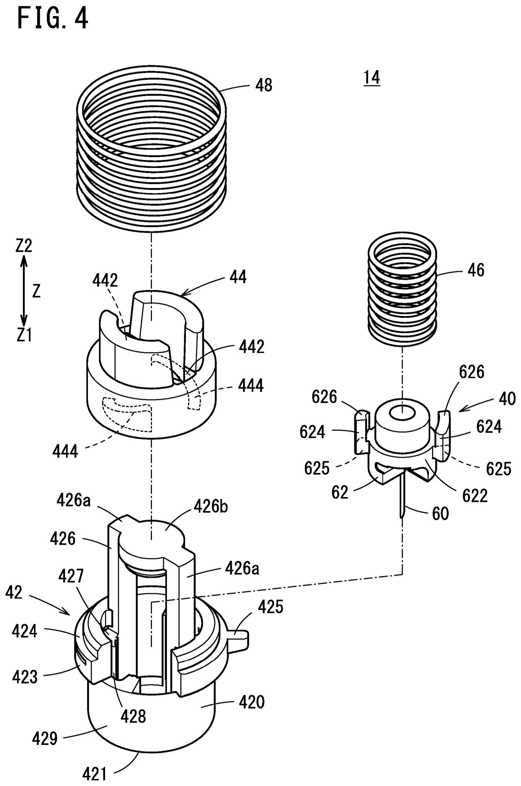

As illustrated in Fig. 4, the needle member 40 includes a puncture needle 60 and a needle hub 62 that holds the puncture needle 60. The needle member 40 is relatively rotatable with respect to the abutting member 42 about an axis of the needle member 40. The puncture needle 60 is a hollow needle and projects from the needle hub 62. A distal end of the puncture needle 60 can puncture the skin S of the patient. The needle hub 62 has a hub main body 622 to which the needle hub 62 is fixed, and at least one guide protrusion 624 protruding radially outward from the hub main body 622. In a case where the needle member 40 punctures the skin S of the patient, an end surface of the hub main body 622 in the Z1 direction comes into contact with the skin S. In the present embodiment, a pair of guide protrusions 624 is disposed on opposite sides to each other in a circumferential direction of the hub main body 622. Each guide protrusion 624 has a latching portion 625 at an end portion thereof in the Z1 direction.

As illustrated in Fig. 3, the abutting member 42 is movably provided in the housing 12 along the thickness direction (Z direction) of the housing 12. The abutting member 42 is inserted into the first opening portion 51 of the housing 12. As illustrated in Fig. 4, the abutting member 42 includes a cylindrical portion 420 having a circular cylinder shape and a gate portion 426 that projects from the cylindrical portion 420 in the Z2 direction. The needle member 40 is disposed inside the cylindrical portion 420. The needle member 40 is relatively movable with respect to the abutting member 42 in an axial direction (Z direction) of the abutting member 42.

As illustrated in Fig. 3, an end portion of the cylindrical portion 420 in the Z1 direction has an abutting surface 421 that can be brought into contact with the skin S of the patient (refer to Fig. 9). The abutting member 42 has a needle lead-out port 422 that is open at the end portion of the cylindrical portion 420 in the Z1 direction. The abutting surface 421 surrounds the needle lead-out port 422. The puncture needle 60 can project from the needle lead-out port 422.

A proximal end of the cylindrical portion 420 has a flange portion 423 having a diameter which increases radially outward. The flange portion 423 has a spring receiving portion 424 having a step shape. The flange portion 423 has an anti-rotation protrusion 425 (refer to Fig. 4). The anti-rotation protrusion 425 engages with a part of the housing 12, thereby preventing the abutting member 42 from rotating with respect to the housing 12.

As illustrated in Fig. 4, the abutting member 42 has at least one engagement step portion 427. The engagement step portion 427 is a flat surface orthogonal to the axial direction of the abutting member 42. In an initial state of the liquid medicine administration device 10 (refer to Fig. 1), the latching portion 625 of the needle hub 62 abuts on the engagement step portion 427 of the abutting member 42. This prevents the needle hub 62 from moving in the puncture direction (Z1 direction) with respect to the abutting member 42.

The cylindrical portion 420 has at least one guide groove 428 that can guide the needle member 40 in the axial direction. In the present embodiment, the cylindrical portion 420 has a pair of guide grooves 428 provided on opposite sides to each other in a circumferential direction thereof. Proximal ends of the guide grooves 428 are individually disposed at positions adjacent to the engagement step portion 427 in the circumferential direction of the cylindrical portion 420. The guide groove 428 is recessed radially outward from an inner circumferential surface of the cylindrical portion 420 and extends along the axial direction of the abutting member 42. The guide groove 428 can receive the guide protrusion 624 of the needle member 40.

As illustrated in Fig. 4, the abutting member 42 has a projecting portion 429 that can project in the puncture direction from the first opening portion 51 of the housing 12. The projecting portion 429 is a part of the cylindrical portion 420. As illustrated in Fig. 4, the gate portion 426 has a pair of column portions 426a extending in the proximal direction from the cylindrical portion 420, and a bridge portion 426b connecting proximal ends of the pair of column portions 426a.

The puncture biasing member 46 is disposed between the hub main body 622 of the needle hub 62 and the bridge portion 426b of the gate portion 426. The puncture biasing member 46 is a spring member including a coil spring. The puncture biasing member 46 elastically biases the needle member 40 in the puncture direction (Z1 direction).

The pusher 44 is disposed inside the housing 12 to be relatively movable with respect to the housing 12 and the abutting member 42 in the axial direction (Z1 direction) of the abutting member 42. The pusher 44 is formed in an annular ring shape. A pair of grooves 442 is provided in an inner circumferential portion of the pusher 44. A pair of column portions 426a of the gate portion 426 engages with the pair of grooves 442. This enables the gate portion 426 to guide the pusher 44 in the axial direction of the abutting member 42 and prevents the pusher 44 from relatively rotating with respect to the abutting member 42.

As illustrated in Fig. 3, in the initial state of the liquid medicine administration device 10, the top portion (proximal end) of the pusher 44 is disposed in the hole portion 123 of the housing 12. The pusher 44 has at least one first inclined portion 444. The first inclined portion 444 is provided on a distal end side of the pusher 44 and faces the distal direction (Z1 direction) of the pusher 44. In the present embodiment, the pusher 44 has a pair of first inclined portions 444 disposed on opposite sides to each other in the circumferential direction. The first inclined portions 444 are individually inclined with respect to the axial direction of the pusher 44.

As illustrated in Fig. 4, the needle hub 62 has at least one second inclined portion 626 that abuts on the first inclined portion 444. The second inclined portion 626 faces the proximal direction (Z2 direction) of the needle hub 62. In the present embodiment, the needle hub 62 has a pair of second inclined portions 626 disposed on opposite sides to each other in the circumferential direction. The pair of second inclined portions 626 is provided at proximal ends of the pair of guide protrusions 624, respectively. The second inclined portion 626 is inclined with respect to the axial direction (Z direction) of the needle member 40 in the same direction as the first inclined portion 444. In the initial state of the liquid medicine administration device 10, the pair of first inclined portions 444 and the pair of second inclined portions 626 face each other.

As illustrated in Fig. 3, the following biasing member 48 is disposed between the cylindrical portion 420 of the abutting member 42 and the ceiling portion 30 of the housing 12 in the housing 12. The following biasing member 48 is a spring member including a coil spring. One end of the following biasing member 48 abuts on the spring receiving portion 424 of the cylindrical portion 420. The other end of the following biasing member 48 abuts on the ceiling portion 30 of the housing 12. The following biasing member 48 elastically biases the abutting member 42 in the puncture direction (Z1 direction).

As illustrated in Fig. 2, a connection needle 70, a coupling tube 71, and a connection cylinder 72 are disposed inside the housing 12. The connection needle 70 is formed in a tubular shape. The connection needle 70 extends along the X direction. A needle tip of the connection needle 70 faces the X2 direction. The connection needle 70 has the needle tip that can penetrate a sealing member 744 to be described below.

The coupling tube 71 is a flexible tube for transferring the liquid medicine from the connection needle 70 to the needle hub 62 (Fig. 4). One end of the coupling tube 71 is connected to the connection needle 70. The other end of the coupling tube 71 is coupled to the needle hub 62. The connection cylinder 72 covers the connection needle 70. The connection cylinder 72 is open in the X2 direction.

The syringe pump 16 includes a prefilled syringe 74 and a drive unit 75. The syringe pump 16 is disposed inside the housing 12 in a slidable state along a longitudinal direction (X direction) of the housing 12.

The prefilled syringe 74 includes a syringe main body 740, a gasket 742, the sealing member 744, and a distal cap 746. The syringe main body 740 is filled with the liquid medicine in advance. The syringe main body 740 is made of a material having transparency. A user can visually recognize the liquid medicine in the prefilled syringe 74 positioned inside the housing 12 via a window 121 provided in the housing 12.

The gasket 742 is made of an elastic resin material such as a rubber material or an elastomer material and is slidably disposed inside the syringe main body 740. The sealing member 744 is made of an elastic resin material such as a rubber material or an elastomer material and liquid-tightly seals a distal opening of the syringe main body 740. The distal cap 746 fixes the sealing member 744 to the distal opening of the syringe main body 740.

The drive unit 75 has a pressing element 750 for pressing the gasket 742 in the distal direction. Although not illustrated in detail, the drive unit 75 further includes a power supply unit (for example, a battery), a motor, a power transmission mechanism, a control unit, or the like.

The needle cover 18 is provided movably with respect to the housing 12. Specifically, the needle cover 18 is supported by the housing 12 to be rotatable around an axis along the width direction of the housing 12. A pair of support shafts 328 is provided respectively on side wall portions 320 constituting both sides of the circumferential wall portion 32 of the housing 12 in the width direction. The pair of support shafts 328 projects from the side wall portions 320 on both sides, respectively. The needle cover 18 is rotatably supported by the pair of support shafts 328.

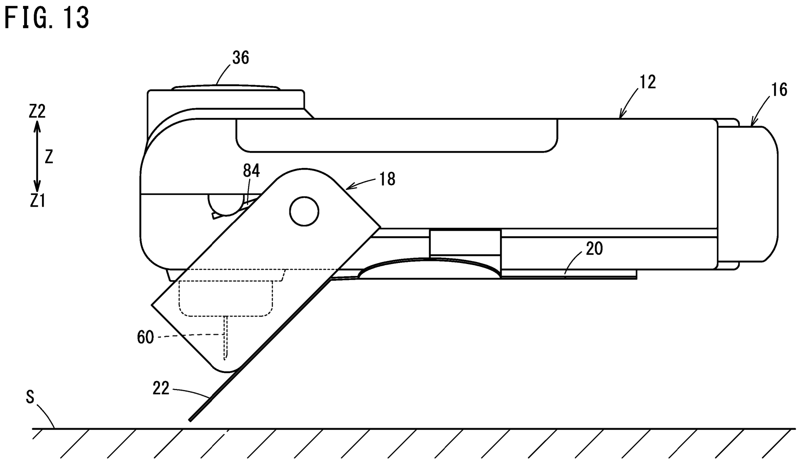

The needle cover 18 is movable from a puncture permission position at which the puncture needle 60 is permitted to puncture the skin S to a shielding position at which the needle tip of the puncture needle 60 is covered. In Fig. 3, the needle cover 18 is located at the puncture permission position. In Fig. 13, the needle cover 18 is located at the shielding position. As illustrated in Fig. 2, the needle cover 18 has a shape of a lid-shaped member that partially covers the housing 12. The needle cover 18 is in a lid closed state at the puncture permission position and is in a lid open state at the shielding position.

The needle cover 18 has a first cover wall 181 facing the bottom wall portion 28 of the housing 12, a second cover wall 182 facing a distal wall of the housing 12, and a pair of third cover walls 183 facing the side wall portions 320 on both sides of the housing 12. Hereinafter, the first cover wall 181, the second cover wall 182, and the pair of third cover walls 183 may be collectively referred to as a "cover wall 180". The cover wall 180 is a portion of the needle cover 18 that is not a hole (opening). The cover wall 180 covers the needle tip of the puncture needle 60 in the lid open state of the needle cover 18.

The needle cover 18 has an opening portion 81(hereinafter, referred to as a first opening portion 81) that permits the puncture structure 14 to be received in the lid closed state (at the puncture permission position). The first opening portion 81 penetrates the first cover wall 181. In a case where the needle cover 18 is located at the puncture permission position, the first opening portion 81 permits the abutting member 42 to be received. A second opening portion 82 is formed in the first cover wall 181.

The needle cover 18 has a notch portion 83 communicating with the first opening portion 81. The notch portion 83 penetrates the first cover wall 181. When the needle cover 18 moves from the puncture permission position to the shielding position, a part of the puncture structure 14 passes through the notch portion 83.

The second cover wall 182 projects in the Z2 direction from a distal end of the first cover wall 181 and extends in the width direction of the housing 12. The pair of third cover walls 183 projects in the Z2 direction from both sides of the first cover wall 181 in the width direction and extend in the proximal direction from both sides of the second cover wall 182 in the width direction. The pair of support shafts 328 of the housing 12 is inserted into support holes 184 provided in the pair of third cover walls 183, respectively.

The liquid medicine administration device 10 further includes a shielding biasing member 84 that biases the needle cover 18 toward the shielding position. In the present embodiment, the shielding biasing member 84 is a torsion spring. The shielding biasing member 84 has a coil portion 840, a first arm portion 841 extending from one end of the coil portion 840, and a second arm portion 842 extending from the other end of the coil portion 840. One support shaft 328 provided in the housing 12 is inserted into the coil portion 840. The first arm portion 841 engages with a first latching protrusion 85 provided on the housing 12. The second arm portion 842 engages with a second latching protrusion 86 provided on the needle cover 18 (specifically, one third cover wall 183). Note that the shielding biasing member 84 is not limited to the form of the torsion spring described above, and may be, for example, a coil spring disposed between the housing 12 and the first cover wall 181.

The sliding member 20 faces the bottom wall portion 28 of the housing 12 and is provided slidably with respect to the housing 12. The sliding member 20 is movable with respect to the housing 12 from a pre-sliding position to a post-sliding position. In Fig. 9, the sliding member 20 is located at the pre-sliding position. In Fig. 13, the sliding member 20 is located at the post-sliding position.

As illustrated in Fig. 2, the sliding member 20 has a sliding plate 200, a first lock portion 210, a second lock portion 220, and a pair of guide arms 230. The sliding plate 200, the first lock portion 210, and the second lock portion 220 face the bottom wall portion 28 of the housing 12. The sliding plate 200 constitutes a main body of the sliding member 20.

The first lock portion 210 engages with a first engagement portion 110 provided in the bottom wall portion 28 of the housing 12. The first engagement portion 110 is a hole penetrating the bottom wall portion 28. The first engagement portion 110 may be a groove provided in the bottom wall portion 28.

The first lock portion 210 is a movable piece provided to be movable in the thickness direction (Z direction) with respect to the sliding plate 200. The first lock portion 210 is movable with respect to the sliding plate 200 in a direction away from the bottom wall portion 28 of the housing 12.

The first lock portion 210 has a first plate 212 and an engagement protrusion 214. A part of a circumferential edge portion of the first plate 212 is connected to the sliding plate 200 via a connection portion 216. In the illustrated example, the connection portion 216 is provided on one side of the circumferential edge portion of the first plate 212 in the width direction (Y direction), and may be provided at any position of the circumferential edge portion of the first plate 212. The sliding plate 200 has a first notch portion 202 formed to surround the first lock portion 210 (the first plate 212) in a C shape. The engagement protrusion 214 projects toward the housing 12. In the initial state of the liquid medicine administration device 10, the engagement protrusion 214 is inserted into the first engagement portion 110.

When the housing 12 is separated from the skin S of the living body, the first lock portion 210 is drawn by the skin S to be separated from the bottom wall portion 28 of the housing 12, and the engagement protrusion 214 and the first engagement portion 110 are disengaged.

The second lock portion 220 is an elastic piece having one end coupled to the sliding plate 200. The sliding plate 200 has a second notch portion 204 formed to surround the second lock portion 220 in a U shape. The second lock portion 220 is displaceable with respect to the sliding plate 200 in the thickness direction (Z direction) of the sliding plate 200. The second lock portion 220 has a second plate 222 and an engagement claw portion 224. The second plate 222 extends from the sliding plate 200 in the proximal direction (X2 direction) of the housing 12. The engagement claw portion 224 projects from a free end of the second plate 222 toward the housing 12. The engagement claw portion 224 has an inclined surface 225 inclined with respect to a moving direction of the sliding member 20.

In relation to the second lock portion 220, the housing 12 has an accommodation portion 124 and a second engagement portion 120. The accommodation portion 124 accommodates the engagement claw portion 224 in a case where the sliding member 20 is located at the pre-sliding position. The accommodation portion 124 is a hole penetrating the bottom wall portion 28 of the housing 12. The accommodation portion 124 may be a groove formed in the bottom wall portion 28.

The second engagement portion 120 engages with the engagement claw portion 224 in a case where the sliding member 20 is located at the post-sliding position. The second engagement portion 120 is a hole penetrating the bottom wall portion 28 of the housing 12. The second engagement portion 120 may be a groove formed in the bottom wall portion 28. In the housing 12, the second engagement portion 120 is formed on the proximal side from the accommodation portion 124.

Note that the accommodation portion 124 and the second engagement portion 120 may be formed at a location other than the bottom wall portion 28 of the housing 12. For example, the accommodation portion 124 and the second engagement portion 120 may be formed in one side wall portion 320 of the housing 12. In this case, the second lock portion 220 is disposed to face one side wall of the housing 12.

The pair of guide arms 230 projects in the Z2 direction from both ends of the sliding plate 200 in the width direction. Protrusions 232 provided on the pair of guide arms 230 engage with groove portions 322 provided in the side wall portions 320 on both sides of the housing 12 in the width direction, respectively. The groove portions 322 extend in the longitudinal direction (X direction) of the housing 12. The protrusions 232 of the pair of guide arms 230 engage with the pair of grooves 322, thereby guiding the sliding member 20 along the longitudinal direction of the housing 12.

The needle cover 18 and the sliding member 20 are coupled via a coupling portion 19 to be linked to each other. The needle cover 18, the coupling portion 19, and the sliding member 20 are formed as one component. That is, the needle cover 18, the coupling portion 19, and the sliding member 20 constitute an integrally molded cover member 17.

The coupling portion 19 extends in the width direction of the housing 12. A distal portion of the coupling portion 19 is coupled to a proximal portion of the needle cover 18. A proximal portion of the coupling portion 19 is coupled to a distal portion of the sliding member 20. A surface of the cover member 17 facing the bottom wall portion 28 of the housing 12 has a first groove 171 and a second groove 172. The first groove 171 is provided at a coupling position between the needle cover 18 and the coupling portion 19 and extends in the width direction of the housing 12. The second groove 172 is provided at a coupling position between the sliding member 20 and the coupling portion 19 and extends in the width direction of the housing 12. The cover member 17 is thin at the positions of the first groove 171 and the second groove 172. Therefore, the cover member 17 can be bent at the positions of the first groove 171 and the second groove 172. Note that, in another aspect, the coupling portion 19 may be formed in a sheet shape with flexibility.

As described above, the needle cover 18 and the sliding member 20 are coupled via the coupling portion 19 to be linked to each other. Therefore, in a case where the needle cover 18 is located at the puncture permission position, the sliding member 20 is located at the pre-sliding position, at which the sliding member 20 is fixed to the bottom wall portion 28 of the housing 12 by the first lock portion 210, and restricts the needle cover 18 from moving to the shielding position. When the housing 12 is separated from the skin S of the living body, the first engagement portion 110 and the first lock portion 210 are disengaged, so that the sliding member 20 is movable to the post-sliding position and the needle cover 18 is movable to the shielding position.

The adhesive member 22 is a flexible sheet-shaped member. The adhesive member 22 is fixed to the needle cover 18 and the sliding member 20. Specifically, the adhesive member 22 is fixed to a surface of the first cover wall 181 on the opposite side to the housing 12. The adhesive member 22 is fixed to a surface of the sliding member 20 on the opposite side to the housing 12. The adhesive member 22 is also fixed to the coupling portion 19. Note that the adhesive member 22 may be fixed to at least the sliding member 20 among the needle cover 18, the coupling portion 19, and the sliding member 20. That is, the adhesive member 22 may not be fixed to at least one of the needle cover 18 and the coupling portion 19.

Although not illustrated in detail, the adhesive member 22 has a base sheet and an adhesive sheet. The base sheet is fixed to the cover member 17. The base sheet is a flexible resin sheet. Examples of a constituent material of the base sheet include, but are not limited to, polyethylene terephthalate (PET).

The adhesive sheet is provided on a surface of the base sheet on the opposite side to the cover member 17. The adhesive sheet is an adhesive surface 23 (bonding surface) that can adhere to the skin S of the living body. A knob portion 92 is provided on an outer circumferential portion of the adhesive member 22. The adhesive surface 23 is not provided on the knob portion 92.

A part of the adhesive member 22 is fixed to the first lock portion 210. Specifically, the adhesive member 22 has a first locking adhesive portion 94 fixed to the first lock portion 210. The adhesive member 22 has a locking notch portion 96 formed to surround the first locking adhesive portion 94 in a C shape. A notch portion 241 having a C shape similar to that of the locking notch portion 96 is also formed in the release sheet 24.

A second locking opening portion 98 is formed in a portion of the adhesive member 22 which corresponds to the second lock portion 220. An opening portion 242 similar to the second locking opening portion 98 is also formed in the release sheet 24. The second lock portion 220 is exposed via the second locking opening portion 98. Therefore, the adhesive member 22 is not fixed to the second lock portion 220.

The adhesive member 22 further has opening portions 101 and 102 and a notch portion 103 which correspond to the first opening portion 81, the second opening portion 82, and the notch portion 83 of the needle cover 18, respectively. Similarly, the release sheet 24 has opening portions 251 and 252 and a notch portion 253 which correspond to the first opening portion 81, the second opening portion 82, and the notch portion 83 of the needle cover 18.

The release sheet 24 releasably adheres to the adhesive surface 23 of the adhesive member 22 in the initial state of the liquid medicine administration device 10. The release sheet 24 is removed from the adhesive surface 23 when the liquid medicine administration device 10 is used. The release sheet 24 adheres to the entire adhesive surface 23.

A knob portion 91 is provided on an outer circumferential portion of the release sheet 24. Hereinafter, the knob portion 91 provided on the release sheet 24 is referred to as a "first knob portion 91". The knob portion 92 provided on the adhesive member 22 is referred to as a "second knob portion 92".

As illustrated in Fig. 1, in the initial state of the liquid medicine administration device 10, the attachment 26 is mounted on the needle cover 18. The attachment 26 is detachable from the needle cover 18. Hereinafter, a state where the attachment 26 is mounted on the needle cover 18 is referred to as a "mounted state of the attachment 26".

As illustrated in Fig. 2, the attachment 26 has an unsealing portion 260, a puncture seal portion 262, and a connection seal portion 264. In the mounted state of the attachment 26, the attachment 26 holds the puncture structure 14 and the prefilled syringe 74 in a sterile condition.

The unsealing portion 260 is made of, for example, a hard resin material. The unsealing portion 260 is formed in a U shape. In the mounted state of the attachment 26, a pair of engagement claws 261 provided at both end portions of the unsealing portion 260 detachably engages with the pair of third cover walls 183 of the needle cover 18. In the mounted state of the attachment 26, a part of the adhesive member 22 is disposed between the unsealing portion 260 and the needle cover 18.

The puncture seal portion 262 is provided on the unsealing portion 260. In the mounted state of the attachment 26, the puncture seal portion 262 is in close contact with the abutting surface 421 of the abutting member 42, airtightly closes the needle lead-out port 422, and seals the puncture needle 60. The connection seal portion 264 is provided on a projecting wall 263 projecting from the unsealing portion 260. In the mounted state of the attachment 26, the connection seal portion 264 is inserted into the second opening portion 52 of the housing 12, airtightly closes an opening portion of the cylindrical portion 420, and seals the connection needle 70.

The liquid medicine administration device 10 is used as follows.

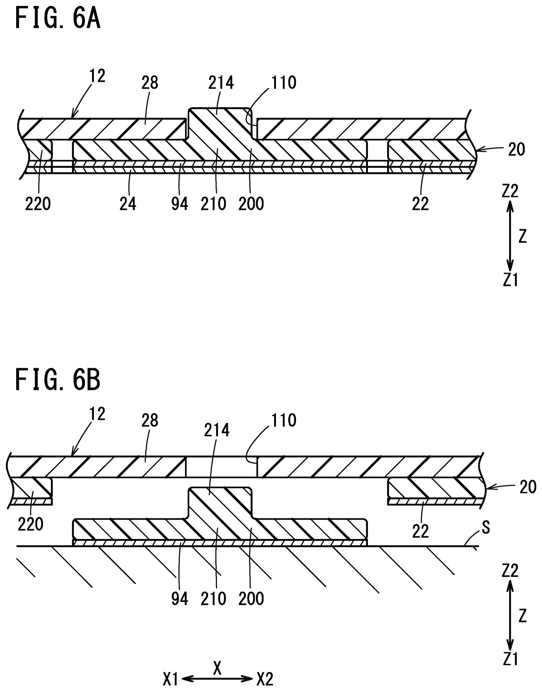

In the initial state of the liquid medicine administration device 10 illustrated in Fig. 1, the first lock portion 210 of the sliding member 20 engages with the first engagement portion 110 of the housing 12 (refer to Fig. 6A). Therefore, the needle cover 18 is restricted from moving to the shielding position, and even if an external force toward the shielding position acts on the needle cover 18, the needle cover 18 is maintained at the puncture permission position which is the initial position.

In the case of using the liquid medicine administration device 10, the user removes the attachment 26 from the needle cover 18. This results in unsealing the puncture needle 60 sealed by the puncture seal portion 262 and unsealing the connection needle 70 sealed by the connection seal portion 264. When the attachment 26 is removed, both end portions of the adhesive member 22 in the width direction which are bent by the attachment 26 are restored to the original shape due to the elastic force. Note that a cut, a groove, or the like may be formed in the base sheet of the adhesive member 22 so that the adhesive member 22 can be easily deformed along the shape of the needle cover 18 (alternatively, the housing 12 or the attachment 26) in the state where the attachment 26 is mounted, and the adhesive member 22 can be easily restored to the original shape when the attachment 26 is removed.

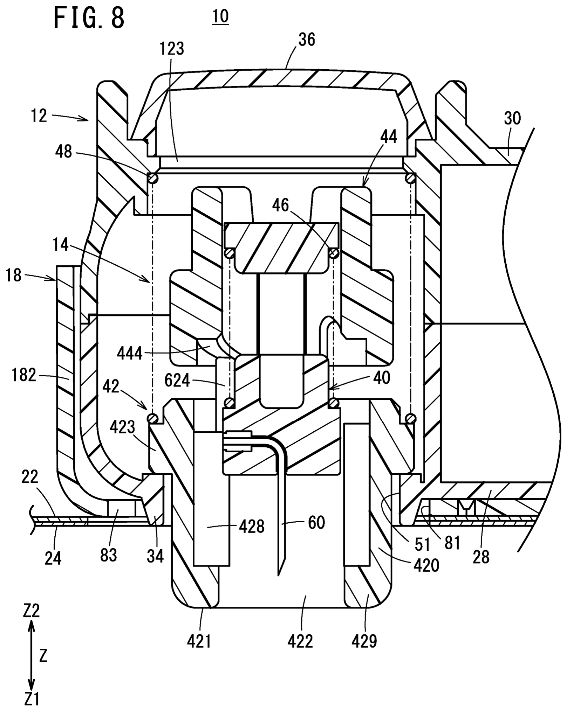

As the attachment 26 is removed, the abutting member 42 is displaced in the Z1 direction with respect to the housing 12 due to an elastic biasing force of the following biasing member 48 as illustrated in Fig. 8. As a result, the cylindrical portion 420 of the abutting member 42 projects in the Z1 direction from the first opening portion 51 of the housing 12. At this time, the flange portion 423 of the abutting member 42 abuts on the bottom wall portion 28 of the housing 12, and the displacement of the abutting member 42 is stopped. Together with the displacement of the abutting member 42, the needle member 40 supported by the abutting member 42 is also displaced in the Z1 direction with respect to the housing 12. At this time, the abutting member 42 and the needle member 40 are not relatively displaced.

Next, the user inserts the syringe pump 16 illustrated in Fig. 2 into the housing 12. This enables the liquid medicine in the prefilled syringe 74 to be sent to the puncture needle 60 since the connection needle 70 penetrates the sealing member 744 of the prefilled syringe 74.

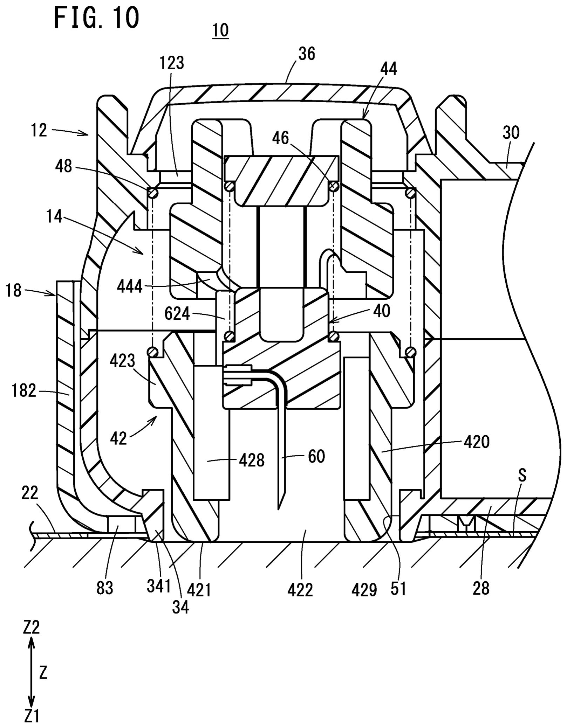

Next, the user pinches the first knob portion 91 with fingers, peels off the release sheet 24 from the adhesive member 22, and exposes the adhesive surface 23 of the adhesive member 22. After peeling off the release sheet 24, the user attaches the adhesive surface 23 to the skin S of the living body as illustrated in Fig. 9. When the adhesive surface 23 adheres to the skin S of the living body, as illustrated in Fig. 10, the abutting member 42 is pushed by the skin S to be displaced in the Z2 direction with respect to the housing 12, with the needle member 40 being taken together, against the biasing force of the following biasing member 48. As a result, the cylindrical portion 420 of the abutting member 42 is housed again in the housing 12, and the abutting surface 421 of the abutting member 42 and a protruding end 341 of the annular protrusion 34 are substantially flush with each other.

Next, the user pushes the pusher 44 in the Z1 direction via the operation cover 36. This results in relatively displacing the pusher 44 with respect to the abutting member 42 in the Z direction. The needle hub 62 rotates about the axis of the needle hub 62 with respect to the abutting member 42 due to the action of the first inclined portion 444 and the second inclined portion 626 according to the relative displacement of the pusher 44 with respect to the abutting member 42 in the Z direction. When the needle hub 62 rotates, and the pair of guide protrusions 624 reaches the pair of guide grooves 428 of the abutting member 42, the pair of guide protrusions 624 is inserted into the pair of guide grooves 428 of the abutting member 42 due to the elastic biasing force of the puncture biasing member 46, and the needle hub 62 is displaced in the Z1 direction (puncture direction).

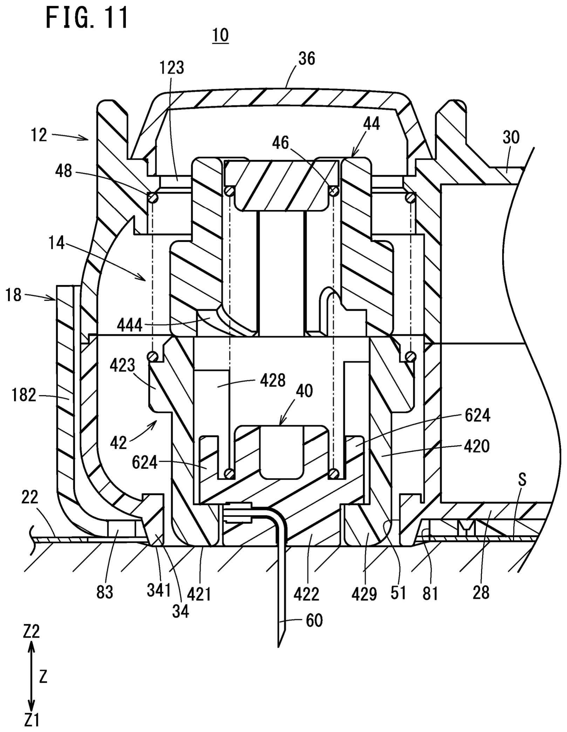

As a result, as illustrated in Fig. 11, the puncture needle 60 punctures the skin S of the patient. In Fig. 11, since the needle cover 18 is held at the puncture permission position, the puncture needle 60 punctures the skin S through the first opening portion 81 of the needle cover 18. Therefore, puncturing of the skin S with the puncture needle 60 is not inhibited by the needle cover 18.

When the user turns on a power supply of the liquid medicine administration device 10 after the puncture of the skin S with the puncture needle 60 is completed, a motor advances the pressing element 750 and the gasket 742 (refer to Fig. 2) via the power transmission mechanism. This results in administering the liquid medicine into the living body.

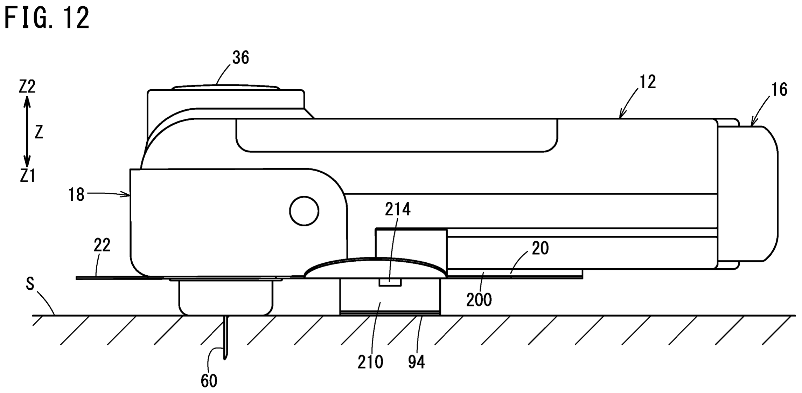

In a case where the administration of the liquid medicine into the living body is ended, and the liquid medicine administration device 10 is removed from the skin S, the second knob portion 92 (Fig. 2) of the adhesive member 22 is pinched by finger, and the adhesive member 22 is pulled up in a direction away from the skin S. At this time, as illustrated in Fig. 12, a portion of the adhesive member 22 other than the first locking adhesive portion 94 is detached from the skin S earlier than the first locking adhesive portion 94. That is, the first locking adhesive portion 94 is detached from the skin S later than the portion of the adhesive member 22 other than the first locking adhesive portion 94.

Since the first locking adhesive portion 94 is fixed to the first lock portion 210, the first lock portion 210 is relatively displaced in the Z1 direction with respect to the sliding plate 200. As a result, as illustrated in Fig. 6B, the engagement protrusion 214 is detached from the first engagement portion 110, and the first lock portion 210 and the first engagement portion 110 are disengaged. As the first lock portion 210 and the first engagement portion 110 are disengaged, the sliding member 20 can be displaced in the proximal direction (X2 direction) with respect to the housing 12.

In addition, the needle cover 18 coupled to the sliding member 20 via the coupling portion 19 can also be relatively displaced with respect to the housing 12. Hence, as the first lock portion 210 and the first engagement portion 110 are disengaged, the needle cover 18 is displaced from the puncture permission position to the shielding position due to the biasing force of the shielding biasing member 84 as illustrated in Fig. 13, and the sliding member 20 is displaced from the pre-sliding position to the post-sliding position. As a result, the puncture needle 60 is covered by the needle cover 18 located at the shielding position. When the needle cover 18 is moved from the puncture permission position to the shielding position, the needle tip of the puncture needle 60 passes through the notch portion 83 (refer to Fig. 2) of the needle cover 18. Therefore, the movement of the needle cover 18 to the shielding position is not hindered by the puncture needle 60.

When the sliding member 20 is displaced from the pre-sliding position to the post-sliding position, the second lock portion 220 of the sliding member 20 moves as follows.

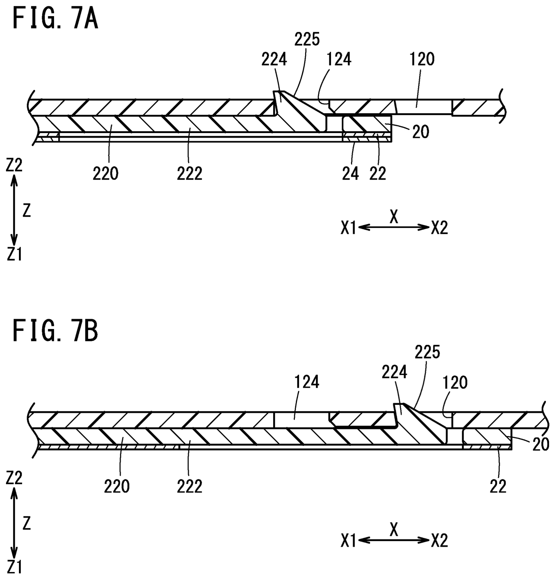

As illustrated in Fig. 7A, in a case where the sliding member 20 is located at the pre-sliding position, the engagement claw portion 224 of the second lock portion 220 is inserted into the accommodation portion 124. When the sliding member 20 starts to be displaced toward the post-sliding position, the inclined surface 225 of the engagement claw portion 224 of the second lock portion 220 comes into sliding contact with the accommodation portion 124, and the second lock portion 220 is elastically deformed in a direction (Z1 direction) away from the accommodation portion 124 of the housing 12. Therefore, the engagement claw portion 224 can be easily detached from the accommodation portion 124.

As illustrated in Fig. 7B, when the sliding member 20 reaches the post-sliding position, the second lock portion 220 is displaced in the Z2 direction due to the elastic restoring force of the second lock portion 220, and the engagement claw portion 224 is inserted into the second engagement portion 120. As a result, the second lock portion 220 engages with the second engagement portion 120 and restricts the sliding member 20 from moving toward the pre-sliding position. That is, the sliding member 20 is fixed to the post-sliding position by the second lock portion 220.

The present embodiment provides the following effects.

As illustrated in Fig. 6A, in the liquid medicine administration device 10, in a case where the needle cover 18 is located at the puncture permission position, the sliding member 20 is located at the pre-sliding position, at which the sliding member 20 is fixed to a bottom wall of the housing 12 by the first lock portion 210, and restricts the needle cover 18 from moving to the shielding position. When the housing 12 is separated from the skin S of the living body, the first engagement portion 110 and the first lock portion 210 are disengaged as illustrated in Fig. 6B, so that the sliding member 20 is movable to the post-sliding position and the needle cover 18 is movable to the shielding position.

As described above, since the movement of the needle cover 18 to the shielding position is restricted by the first lock portion 210 in the initial state of the liquid medicine administration device 10, the needle tip of the puncture needle 60 is not covered with the needle cover 18, and the puncture of the skin S of the living body can be performed. On the other hand, since the movement restriction of the needle cover 18 by the first lock portion 210 is cancelled by separating the housing 12 from the skin S of the living body after the administration of the liquid medicine, the needle tip of the puncture needle 60 can be covered with the needle cover 18.

A part of the adhesive member 22 (the first locking adhesive portion 94) is fixed to the first lock portion 210. When the housing 12 is separated from the skin S of the living body, the first lock portion 210 is drawn by the skin S to be separated from the bottom wall portion 28 of the housing 12 and is disengaged from the first engagement portion 110. This enables, in a simple structure, a motion of the housing 12 that is separated from the skin S of the living body and the disengagement of the first lock portion 210 and the first engagement portion 110 from each other to be linked to each other.

The first lock portion 210 is a movable piece provided to be movable with respect to the sliding plate 200 of the sliding member 20. A part of the circumferential edge portion of the first lock portion 210 is coupled to the sliding plate 200. This enables the first lock portion 210 to be separated from the first engagement portion 110 in a simple configuration when the housing 12 is separated from the skin S of the living body.

As illustrated in Fig. 7A, the sliding member 20 has the second lock portion 220 that is engageable with the second engagement portion 120 provided in the housing 12. In a case where the sliding member 20 is located at the post-sliding position, the second lock portion 220 engages with the second engagement portion 120 and restricts the sliding member 20 from moving toward the pre-sliding position. According to this configuration, since the sliding member 20 is fixed to the post-sliding position by the second lock portion 220, the needle cover 18 can be prevented from returning to the puncture permission position.

The second lock portion 220 is an elastic piece having one end coupled to the sliding plate 200 of the sliding member 20. This enables, in a simple structure, the second lock portion 220 to engage with the second engagement portion 120 of the housing 12 in a case where the sliding member 20 reaches the post-sliding position.

The second lock portion 220 has the engagement claw portion 224 having the inclined surface 225. The housing 12 has the accommodation portion 124 that accommodates the engagement claw portion 224 in a case where the sliding member 20 is located at the pre-sliding position. When the sliding member 20 moves from the pre-sliding position toward the post-sliding position, the inclined surface 225 comes into sliding contact with the accommodation portion 124, and the second lock portion 220 is deformed in the direction away from the accommodation portion 124, so that the engagement claw portion 224 is detached from the accommodation portion 124. According to this configuration, since the engagement claw portion 224 of the second lock portion 220 has the inclined surface 225, the engagement claw portion 224 can be smoothly detached from the accommodation portion 124.

The adhesive member 22 is not fixed to the second lock portion 220. According to this configuration, the second lock portion 220 can be prevented from being drawn and deformed by the skin S when the adhesive member 22 is detached from the skin S.

As illustrated in Fig. 2, the needle cover 18 has a shape of a lid-shaped member which partially covers the housing 12, is in the lid closed state at the puncture permission position, and is in the lid open state at the shielding position. The needle cover 18 has the opening portion 81 that permits reception of the puncture structure 14 in the lid closed state, and the cover wall 180 that covers the needle tip of the puncture needle 60 in the lid open state. According to this configuration, the needle cover 18 that does not inhibit activation of the puncture structure 14 in the case of the lid closed state and suitably covers the needle tip of the puncture needle 60 in the case of the lid open state can be realized in a simple structure.

As illustrated in Fig. 3, the puncture structure 14 has the abutting member 42 provided movably with respect to the housing 12. The puncture needle 60 is relatively movable with respect to the abutting member 42 and can project from the abutting surface 421 of the abutting member 42 toward the skin S. When the relative positions of the housing 12 and the portion of the skin S which faces the abutting surface 421 change in a state where the housing 12 is placed on the skin S, the abutting member 42 relatively moves with respect to the housing 12 to maintain the contact between the abutting surface 421 and the skin S.

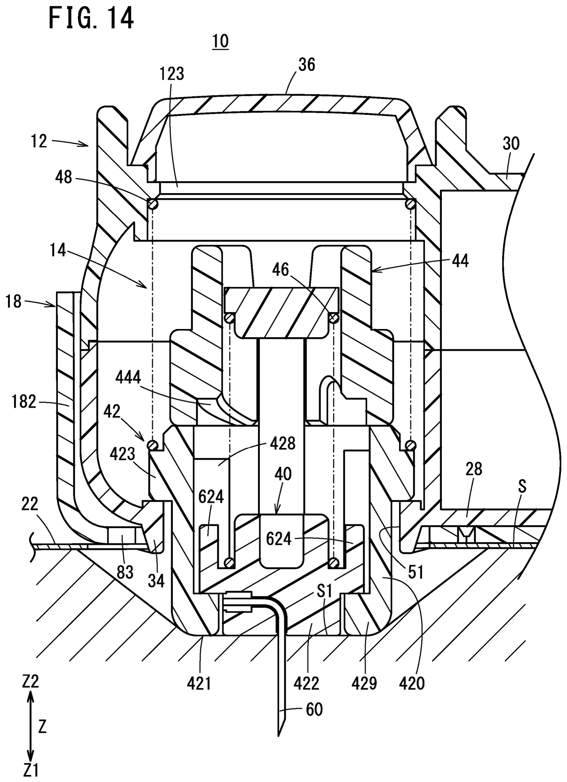

Therefore, as illustrated in Fig. 14, in a case where a facing portion S1 of the skin S is separated from the housing 12 and the needle cover 18 in the Z1 direction, the abutting member 42 biased in the Z1 direction by the following biasing member 48 moves relatively with respect to the housing 12 to maintain the contact of the abutting surface 421 with the facing portion S1. Hence, even in a case where the relative positions of the housing 12 and the skin S change due to a body movement of the living body, in a state where the skin S of the living body is punctured with the puncture needle 60, the abutting member 42 relatively moves with respect to the housing 12 to follow the skin S. Therefore, the puncture needle 60 is appropriately prevented from being pulled out of the skin S, and the puncture of the skin S by the puncture needle 60 is suitably maintained.

As illustrated in Fig. 2, the needle cover 18 has the notch portion 83 communicating with the first opening portion 81. When the needle cover 18 moves from the puncture permission position to the shielding position, a part of the puncture structure 14 passes through the notch portion 83. According to this configuration, the needle cover 18 can be moved to the shielding position without any hindrance by providing the notch portion 83 while an area of a wall portion of the needle cover 18 is sufficiently secured to suitably cover the needle tip of the puncture needle 60.

The liquid medicine administration device 10 further includes the shielding biasing member 84 that biases the needle cover 18 toward the shielding position. According to this configuration, the needle cover 18 can be automatically moved to the shielding position by the biasing force of the shielding biasing member 84 when the first lock portion 210 and the first engagement portion 110 are disengaged.

The needle cover 18, the coupling portion 19, and the sliding member 20 are formed as one component. According to this configuration, an increase in the number of components can be reduced.

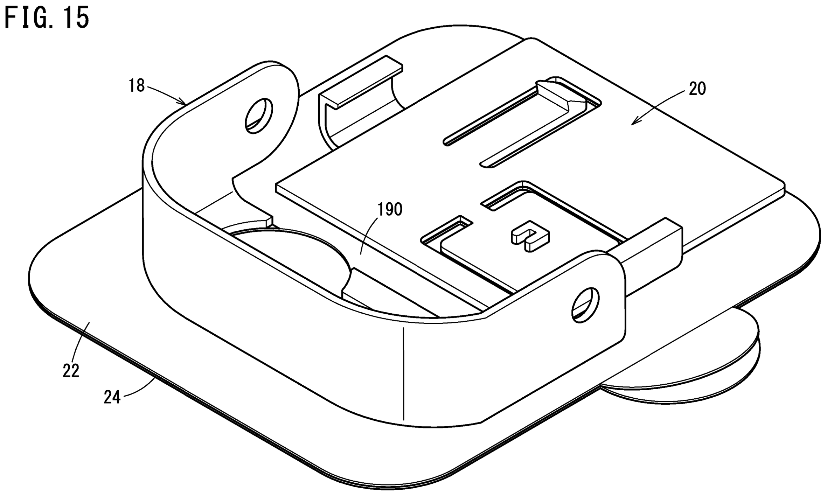

Note that the needle cover 18, the coupling portion 19, and the sliding member 20 do not have to be formed as one component. For example, in the configuration illustrated in Fig. 15, a coupling portion 190 according to a modification example is a part of the adhesive member 22. The adhesive member 22 is fixed to the needle cover 18 and the sliding member 20. In this configuration, the needle cover 18 and the sliding member 20 are independent components of each other. The needle cover 18 and the sliding member 20 are coupled via the coupling portion 190 that is a part of the adhesive member 22. Therefore, the motion of the needle cover 18 and the motion of the sliding member 20 with respect to the housing 12 can be linked.

Note that the present invention is not limited to the above disclosure and can take various configurations without departing from the gist of the present invention.

## Claims

1. A liquid medicine administration device comprising:
a puncture structure that is capable of puncturing a skin of a living body and has a puncture needle for administering a liquid medicine;
a housing that accommodates the puncture structure and has a bottom wall portion which enables the puncture needle to project;
a needle cover provided movably with respect to the housing;
a sliding member that faces the bottom wall portion of the housing and is provided slidably with respect to the housing; and
an adhesive member that is fixed to the sliding member and has an adhesive surface which is capable of adhering to the skin of the living body, wherein
the needle cover is movable from a puncture permission position at which the puncture needle is permitted to puncture the skin to a shielding position at which a needle tip of the puncture needle is covered,
the needle cover and the sliding member are coupled via a coupling portion to be linked to each other,
the sliding member has a first lock portion that engages with a first engagement portion provided in the housing,
in a case where the needle cover is located at the puncture permission position, the sliding member is located at a pre-sliding position, at which the sliding member is fixed to the housing by the first lock portion, and restricts the needle cover from moving to the shielding position, and
when the housing is separated from the skin of the living body, the first engagement portion and the first lock portion are disengaged, so that the sliding member is movable to a post-sliding position and the needle cover is movable to the shielding position.

2. The liquid medicine administration device according to claim 1, wherein
a part of the adhesive member is fixed to the first lock portion, and
when the housing is separated from the skin of the living body, the first lock portion is drawn by the skin to be separated from the bottom wall portion of the housing and disengaged from the first engagement portion.

3. The liquid medicine administration device according to claim 2, wherein
the sliding member has a sliding plate coupled to the needle cover via the coupling portion,
the first lock portion is a movable piece provided movably with respect to the sliding plate, and
a part of a circumferential edge portion of the first lock portion is coupled to the sliding plate.

4. The liquid medicine administration device according to claim 1, wherein
the sliding member has a second lock portion that is engageable with a second engagement portion provided in the housing, and
in a case where the sliding member is located at the post-sliding position, the second lock portion engages with the second engagement portion and restricts the sliding member from moving toward the pre-sliding position.

5. The liquid medicine administration device according to claim 4, wherein
the sliding member has a sliding plate coupled to the needle cover via the coupling portion, and
the second lock portion is an elastic piece having one end coupled to the sliding plate.

6. The liquid medicine administration device according to claim 5, wherein
the second lock portion has an engagement claw portion having an inclined surface inclined with respect to a moving direction of the sliding member,
the housing has an accommodation portion formed by a groove or a hole that accommodates the engagement claw portion in a case where the sliding member is located at the pre-sliding position,
the second engagement portion is a groove or a hole that engages with the engagement claw portion in a case where the sliding member is located at the post-sliding position, and
when the sliding member moves from the pre-sliding position toward the post-sliding position, the inclined surface comes into sliding contact with the accommodation portion, and the second lock portion is deformed in a direction away from the accommodation portion, so that the engagement claw portion is detached from the accommodation portion.

7. The liquid medicine administration device according to claim 6, wherein the adhesive member is not fixed to the second lock portion.

8. The liquid medicine administration device according to claim 1, wherein
the needle cover has a shape of a lid-shaped member which partially covers the housing, is in a lid closed state at the puncture permission position, and is in a lid open state at the shielding position, and
the needle cover has an opening portion that permits reception of the puncture structure in the lid closed state, and a cover wall that covers the needle tip of the puncture needle in the lid open state.

9. The liquid medicine administration device according to claim 8, wherein
the puncture structure has an abutting member having an abutting surface that comes into contact with the skin,
the abutting member is provided movably with respect to the housing to enable the abutting surface to project from the bottom wall portion,
the opening portion permits the abutting member to be received in a case where the needle cover is located at the puncture permission position,
the puncture needle is relatively movable with respect to the abutting member and is capable of projecting from the abutting surface toward the skin, and
the abutting member relatively moves with respect to the housing to maintain contact between the abutting surface and the skin when relative positions of the housing and a portion of the skin which faces the abutting surface change in a state where the housing is placed on the skin.

10. The liquid medicine administration device according to claim 8, wherein
the needle cover has a notch portion that communicates with the opening portion, and
a part of the puncture structure passes through the notch portion when the needle cover moves from the puncture permission position to the shielding position.

11. The liquid medicine administration device according to any one of claims 1 to 10, comprising a shielding biasing member that biases the needle cover toward the shielding position.

12. any one of claims 1 to 10, wherein the needle cover, the coupling portion, and the sliding member are formed as one component.

13. any one of claims 1 to 10, wherein
the adhesive member is fixed to the needle cover and the sliding member, and
the coupling portion is a part of the adhesive member.
